# EUROPEAN PATENT APPLICATION

(11) **EP 2 604 624 A1**
(43) Date of publication of application: **19.06.2013**
(21) Application number: 11193316.4
(22) Date of filing: 13.12.2011
(51) Int. Cl.: C07K 14/81, C07K 16/38, C07K 7/08

(54) **Bowman-Birk protease Inhibitor (BBI) binding peptides, and uses thereof in the separation, detection or purification of BBI.**

(71) Applicant: University College Dublin National University Of Ireland, Dublin, Dublin 4 (IE)
(72) Inventor: Lee, Gil, Dublin, D4 (IE); Muzard, Julien, Dublin, D4 (IE); Fields, Conor, Dublin, D4 (IE)
(74) Representative: Purdylucey Intellectual Property

(57) **Abstract**

Peptide probes against the soybean Bowman-Birk Inhibitor (BBI) are described. Also described are uses of the peptide probes of the invention for detecting, separating, or purifying BBI from complex protein mixtures such as soy whey, and as an affinity separation matrix comprising a peptide probe of the invention immobilised to a support such as a magnetic bead.

## Description

### Field of the Invention

The present invention relates to Bowman-Birk protease Inhibitor (BBI) binding peptides, and uses thereof in the separation, detection or purification of BBI.

### Background to the Invention

The soybean derived Bowman-Birk Inhibitor (BBI) has long been recognised as an anticancer agent with abundant epidemiological evidence pointing at diets rich in soybean-derived products resulting in lower incidences of cancer. Areas in Asia, especially Japan, have particularly low cancer mortality rates. This has been attributed to the large-scale consumption of soybean products in this region. The suppressive effects of BBI on the carcinogenic process were initially described in 1983 and have since demonstrated similar effects across different species and multiple organ systems. BBI has also been implicated in the treatment of multiple sclerosis, where daily oral administration of a concentrated form of BBI, BBIC, has consistently suppressed multiple sclerosis in animal models. Unsurprisingly, in 1992, BBI gained Investigational New Drug status from the Food and Drug Administration (FDA) and later entered human trial stages, boasting promising results with little toxicity. Soybean proteases account for 6 % of the total protein content of soybeans attributed largely, to the two main inhibitors; BBI and Kunitz trypsin inhibitor (KTI). Multiple BBI isoforms have been described in soybean cultivars, each differing in molecular weight and the extent of interaction with their substrates. The Bowman-Birk inhibitor is a serine protease inhibitor with a molecular weight of 7.8 kDa. Its 71 amino acids are cross linked by seven, conserved disulfide-bridges, making it a robust protein, allowing it to withstand digestive system acidity and remain stable at 100°C for up to 10 min. The double-headed protein contains distinct, kinetically independent antitryptic and antichymotryptic domains with which BBI can form a 1:1 complex or a ternary complex with both substrates. Each domain comprises a conserved motif of three β-strands and evidence suggests that divorcing these domains by scission yields two active components. Specifically, BBI binds to trypsin and/or chymotrypsin via a conserved, exposed protease binding loop observed in all BBI variants. The surface-exposed loop, constrained by the presence of cysteine residues, is complimentary to the protease active site and resides here to inhibit protease activity. This inhibition is believed to account for the anticancer properties of the Bowman-Birk inhibitor. Despite knowledge of its substrates, the exact mechanism by which BBI elicits its anticancer effects remains a mystery. Mouse monoclonal antibodies against BBI have been raised laboriously using the hybridoma approach, allowing quantitative determination of BBI in soy protein mixtures but the lack of probes impedes progress. To this date, tedious and time consuming, multi-step chromatographic procedures have been the convention for BBI purification. In addition, no affinity-based peptide ligands for BBI have been identified, making the isolation and consequently the study of this protein difficult.

It is an objective of the present invention to overcome at least some of the above-mentioned problems.

### Summary of the Invention

The invention is based on the surprising discovery that a number of peptides specifically target the soybean Bowman-Birk Inhibitor (BBI), and thereby facilitate the detection, purification, and/ or isolation of the BBI from a complex protein mixture.

According to the invention, a synthetic peptide probe structure targeting the Bowman-Birk Inhibitor (BBI) is provided, comprising an amino acid sequence (from N to C terminal) selected from the group consisting of:
GAMHLPWHMGTL (SEQ ID NO:1);
KFLGPLNHRDRH (SEQ ID NO:2),
ANYFLPPVLSSS (SEQ ID NO:3),
GAKHLPRHKGPL (SEQ ID NO:4),
GAMHLLRHMGPL (SEQ ID NO:5)
TGHQSPGAYAAH (SEQ ID NO:6),
FTTEDFYNHFPH (SEQ ID NO:7),
APQDNTIMPSPT (SEQ ID NO:8),
MPAVMSSAQVPR (SEQ ID NO:9),
HDFRHSGHNHPP (SEQ ID NO: 10)
DHASHWMVKRGV (SEQ ID NO:11),
SSMYTYHRTGDF (SEQ ID NO:12),
CGLPPHRTC (SEQ ID NO:13),
EVQLQQSGAELVKPGASVKLSCTGSGFNIKDNNIHWLRQRPEQGLEWIG KIDPANGDTEYGPKLQGKATITSDTSSNTAYLQLNSLTSEDTAVYYCAT DLYSKPVWFAYWGQGTLVTVSA (SEQ ID NO: 14),
DVLMSQAPLSLPVSLGDQASISCRSSQNIVHSNGNTYLEWYLQKPGQSP KLLIYKVSNRFSGVPDRFSGRGSGTDFTLKISRVEAEDLGVYYCFQGSHV PWTFGGGTKLEIK (SEQ ID NO: 15),
FNIKDNNIHWLR (SEQ ID NO: 16),
GDTEYGPKLQ (SEQ ID NO: 17),
VWFAYWGQGTLVT (SEQ ID NO: 18),
QNIVHSNGNTYL (SEQ ID NO: 19),
VSNRFSGVPD (SEQ ID NO: 20),
WTFGGGTKLEIK (SEQ ID NO: 21)

Suitably, the peptide includes or consists of the amino acid sequence of any of SEQ ID NO:1 to 13.

In one embodiment, the peptide includes, or consists of, the amino acid sequence of SEQ ID NO:1, 2 or 3.

In a preferred embodiment, the peptide includes or consists of, the amino acid sequence of SEQ ID NO:1.

The peptide probe of the invention may be provided in monomeric or multimeric forms, or may be provided in a bi-, tri-, tetra//quadri-, octo- multivalent structure formats as well as chemically or genetically fused to a carrier or tracer molecular entities (for example biotin, HRP).

Thus, in one preferred embodiment, the invention provides multimeric peptide probe (P)n against the Bowman-Birk Inhibitor (BBI), wherein P is a peptide that consist of an amino acid sequence selected from SEQ ID NO: 1-13, and n = 2-10. Thus, the invention provides a peptide probe against the Bowman-Birk Inhibitor (BBI), comprising an amino acid sequence selected from the group consisting of:
[GAMHLPWHMGTL]n;
[KFLGPLNHRDRH]n;
[ANYFLPPVLSSS]n;
[GAKHLPRHKGPL]n;
[GAMHLLRHMGPL]n;
[TGHQSPGAYAAH]n;
[FTTEDFYNHFPH]n;
[APQDNTIMPSPT]n;
[MPAVMSSAQVPR]n;
[HDFRHSGHNHPP]n;
[DHASHWMVKRGV]n;
[CGLPPHRTC]n; and
[SSMYTYHRTGDF]n,
in which n is selected from 2, 3, 4, 5, 6, 7, 8, 9, 10, 11, 12, preferably 2-10, more preferably 4-8.

The invention also relates to a nucleic acid encoding a peptide of SEQ ID NO: 1 to 21, or a multimeric peptide indicated above.

The peptide probes of the invention (in momomeric, multimeric or multivalent form) may be derivatised (either genetically or chemically) with a linker that is suitable for linking the peptide to another molecule, for example a support or a coupling ligand. In one embodiment of the invention, the linker is a short peptide of typically 1-10 amino acids, preferably 1-6 amino acids, and ideally includes the sequence GGGS (SEQ ID NO: 22) or GGGSCG (SEQ ID NO: 23). Exemplary linkers include GGGS-1,2-diaminoethane and GGGSCG-1,2-diaminoethane.

The peptide probes of the invention (in momomeric, multimeric or multivalent form) may be derivatised (either genetically or chemically) with a detectable label, examples of which are described below.

The invention also provides an affinity separation matrix comprising a support having a peptide probe of the invention (in monomeric or polymeric form) immobilized thereon. Suitably, the support is selected from a plate (i.e. a microtitre plate), a membrane, a bead, a nanoparticle, a magnetic nanoparticle, and a stationary phase of a chromatography column. Suitably, the support is coated with a hydrophilic polymer, for example a poly (ethylene glycol) or a dextran, generally having a molecular weight in the 1,000 to 1,000,000 Da range. Suitably, the hydrophilic polymer is chemically activated to enable immobilisation of peptide.

The invention also relates to a method of preparing an affinity support matrix of the invention, the method comprising a step of providing a support carrying a thin layer of hydrophilic polymer, optionally chemically activating the hydrophilic polymer to enable immobilisation of peptide, and reacting the coated support with a peptide probe of the invention. The invention also provides a Bowman-Birk Inhibitor (BBI) targeting conjugate comprising a peptide of the invention non-covalently or covalently bound to coupling ligand. Examples of suitable coupling ligands will be well known to those skilled in the art and include biotin, peroxydase or poly-Histidine tags.

The invention also provides a kit for detecting or purifying BBI comprising a support having a peptide probe or conjugate of the invention immobilised thereon

The invention also relates to a method of detecting BBI, separating or isolating or purifying BBI from a sample containing, or suspected of containing, BBI, which method employs an affinity separation means comprising a peptide probe of the invention, optionally derivatised with a linker or functional group, coupled to a solid support (typically a magnetic particle or bead), the method comprising the steps of incubating the affinity separation means with the sample for a period of time sufficient to allow the peptide probe bind to any BBI present in the sample, and separation of the affinity separation means from the sample, and optionally eluting the BBI from the affinity separation means.

The invention also relates to an affinity separation means comprising a peptide probe of the invention coupled to a solid support (typically a magnetic particle or bead).

The invention also provides an antibody or antibody fragment having a variable heavy (VH) region, the VH region having an amino acid sequence selected from: a sequence that has greater than 90% sequence identity with the amino acid sequence SEQ ID NO: 14; or the sequence of SEQ ID NO's 16, 17 or 18.

The invention also provides an antibody or antibody fragment having a variable light (VL) region, the VL region having an amino acid sequence selected from: a sequence that has greater than 90% sequence identity with the amino acid sequence SEQ ID NO: 15; or the sequence of SEQ ID NO's 19, 20 or 21.

The invention also relates to the use of a peptide probe, in monomeric, multimeric or multivalent form, optionally derivatised with a linker L, or a conjugate, of the invention for the detection, isolation, separation or purification of soybean BBI protein.

Specific derivatised peptide probes employed in examples below include:
BBI-1: GAMHLPWHMGTLGGGS-1,2-diaminoethane-biotin (>90% purity) - SEQ ID NO: 24
BBI-2: ANYFLPPVLSSSGGGS-1,2-diaminoethane-biotin (>90% purity) - SEQ ID NO: 25
BBI-3: KFLGPLNHRDRHGGGS-1,2-diaminoethane-biotin (>90% purity) - SEQ ID NO: 26
BBI-1C: GAMHLPWHMGTLGGGSCG (>90% purity) - SEQ ID NO: 27
BBI-2C: ANYFLPPVLSSSGGGSCG (>90% purity) - SEQ ID NO: 28
BBI-3C: KFLGPLNHRDRHGGGSCG (>90% purity) - SEQ ID NO: 29

### Brief Description of the Drawings

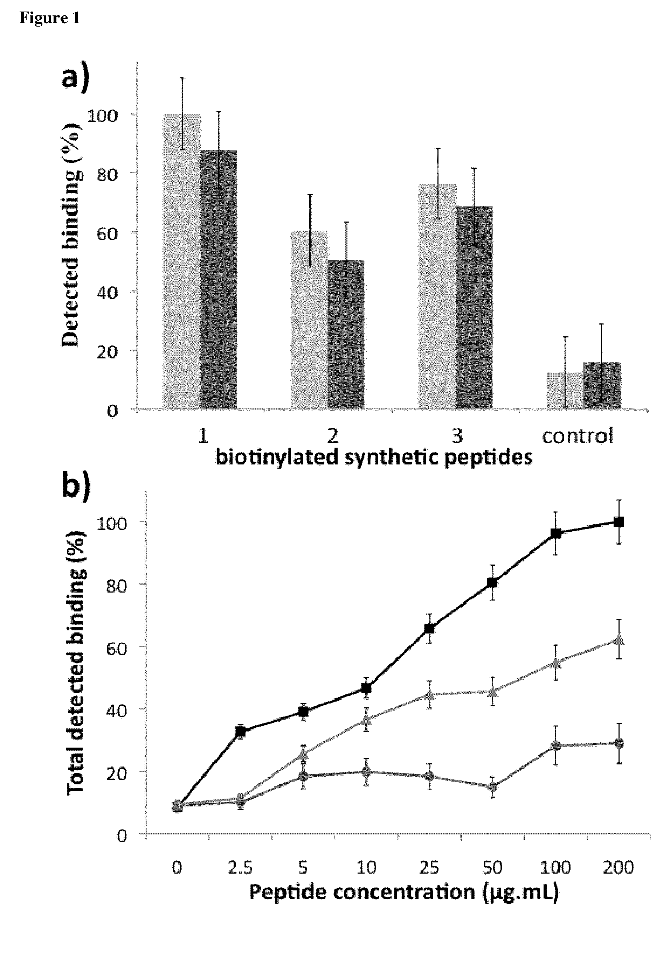

The invention will be more clearly understood from the following description of an embodiment thereof, given by way of example only, with reference to the accompanying drawings, in which:-
**Figure 1****. Synthetic peptide screening.** a) BBI binding ability of each of the three designed synthetic peptide constructs (1 - SEQ ID NO: 24), (2 - SEQ ID NO: 25) and (3 - SEQ ID NO: 26) and control peptides were tested by solid-phase assay procedures using immobilized BBI (light grey) or soy proteins extract - (total protein extracted from soybean) (dark grey). b) Dose-dependent binding of the synthetic biotinylated peptide 1 to BBI (black squares), soy whey (light grey triangles) and soy pulp (grey circles).

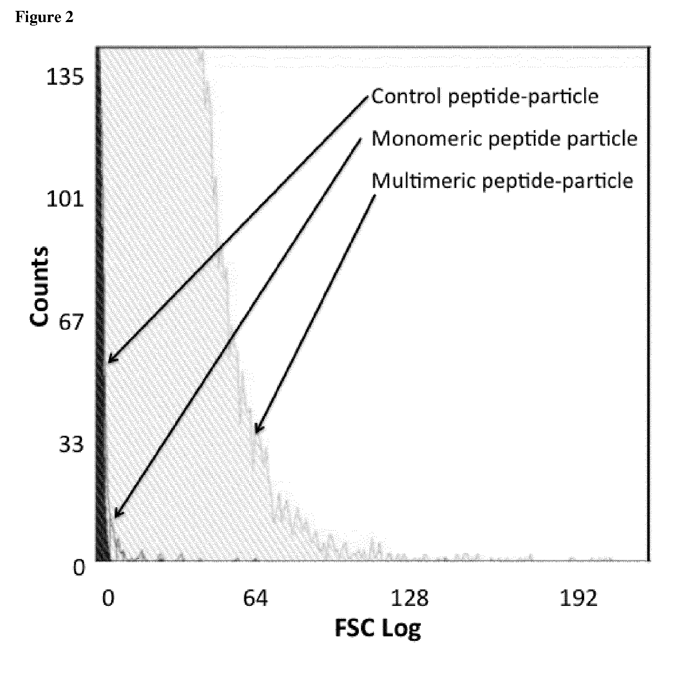

**Figure 2****: Flow cytometry analysis of Cy5-labelled BBI bound to monomeric and multimeric peptide-bound magnetic particles.** Monomeric peptide-bound beads are capable of binding a very small quantity of labelled BBI whereas multimeric peptide-bound beads can bind large quantities.

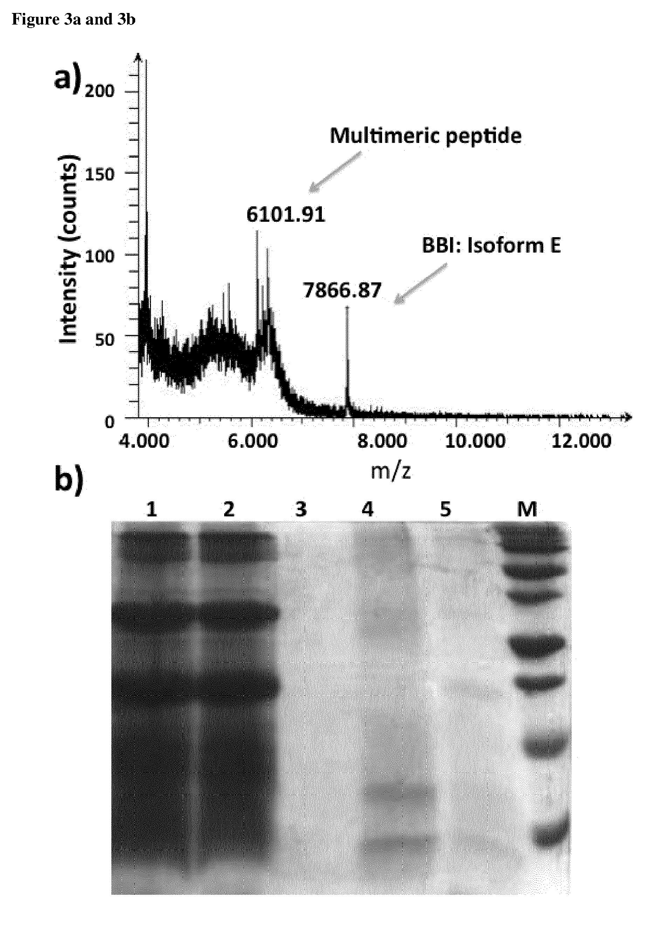

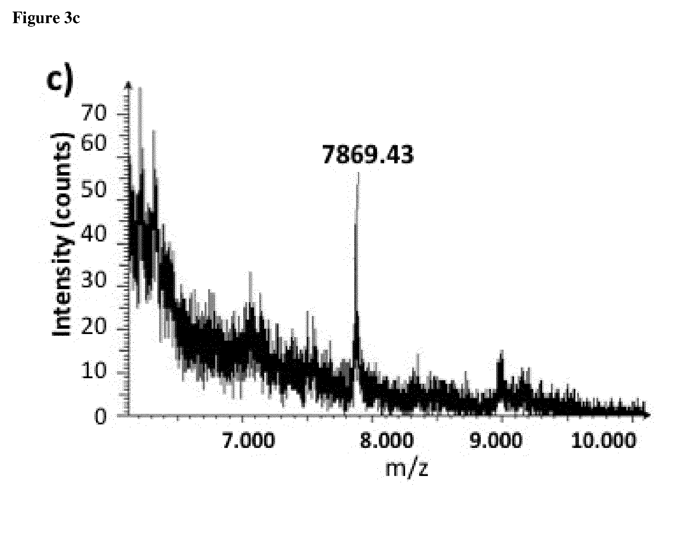

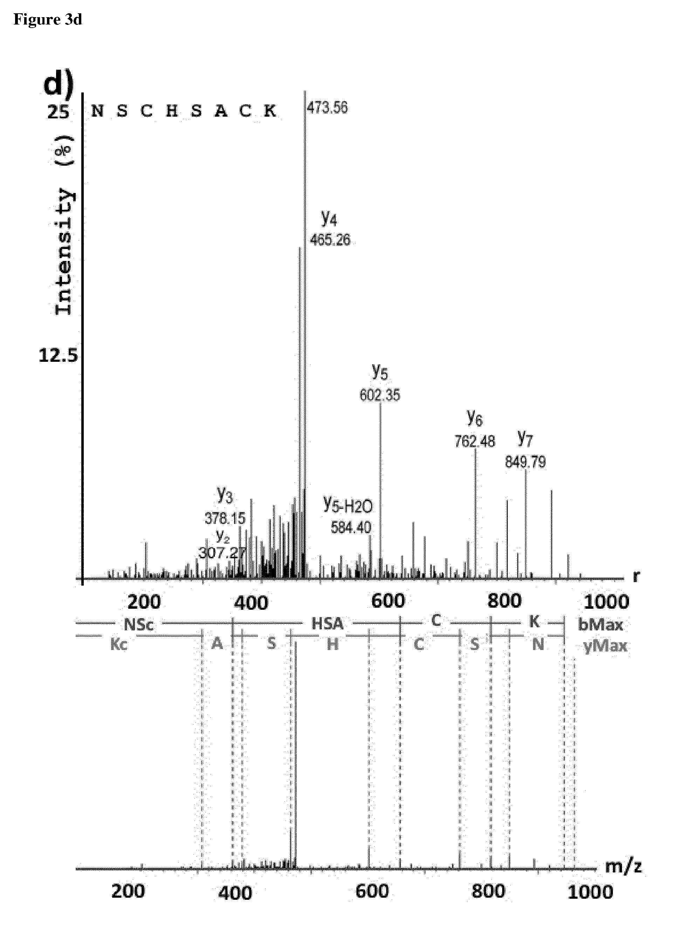

**Figure 3****: Magnetic capture and purification of soybean BBI,** a) direct MALDI-TOF spectrum onto magnetic particles functionalized with the synthetic multimeric peptide with the covalently bound multimeric peptide (SEQ ID 30 and captured soybean BBI. b) SDS-PAGE analysis of eluted fractions. Lane 1: Crude soy whey sample, Lane 2: Non-adsorbed fraction, Lane 3: Final wash, Lane 4: First elution using multimeric BBI-1 peptide-covered particles, Lane 5: First elution using a construct not described here, Molecular weight standards c) MALDI-TOF of the first eluted fraction after incubation of multimeric peptide-bound beads with crude, unprocessed BBI. The resulting peak is within +/- 3 m/z of the expected m/z ratio for purified BBI. d) Tandem MS spectrum of the peptide NSCHSACK from the BBI (Uniprot P01063). Upper panel, matched y-ions are marked in red and annotated. Intensity of spectral lines is 4x magnified respect to base peak intensity. Lower panel, de novo sequencing of the same MS/MS spectrum. Uniprot P01064 (Bowman-Birk type proteinase inhibitor D-II) was also found in eluted fraction from the peptide-particles

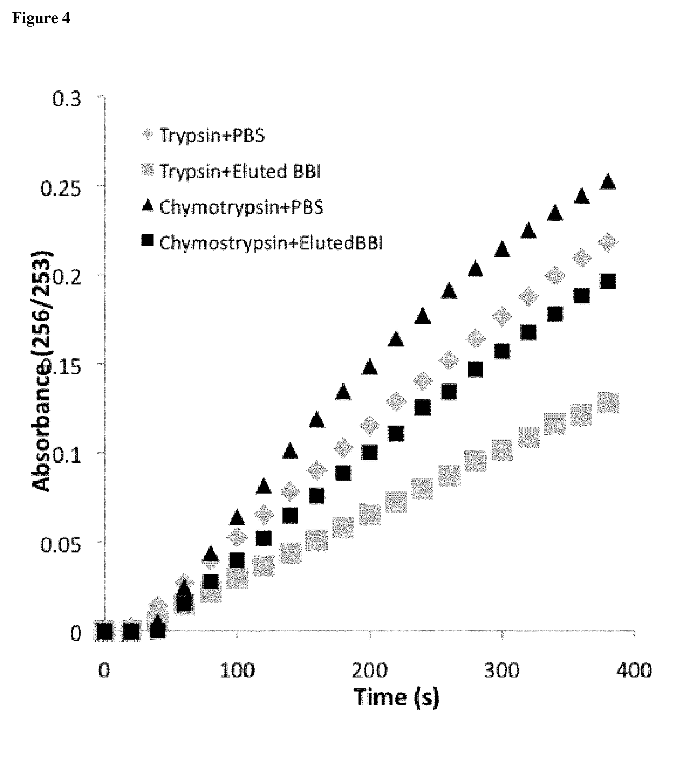

**Figure 4****: The Eluted protein is functional.** Enzymatic (trypsin, grey) and chymotrypsin (black) activities of the eluted fraction bound to the functionalized magnetic particles.

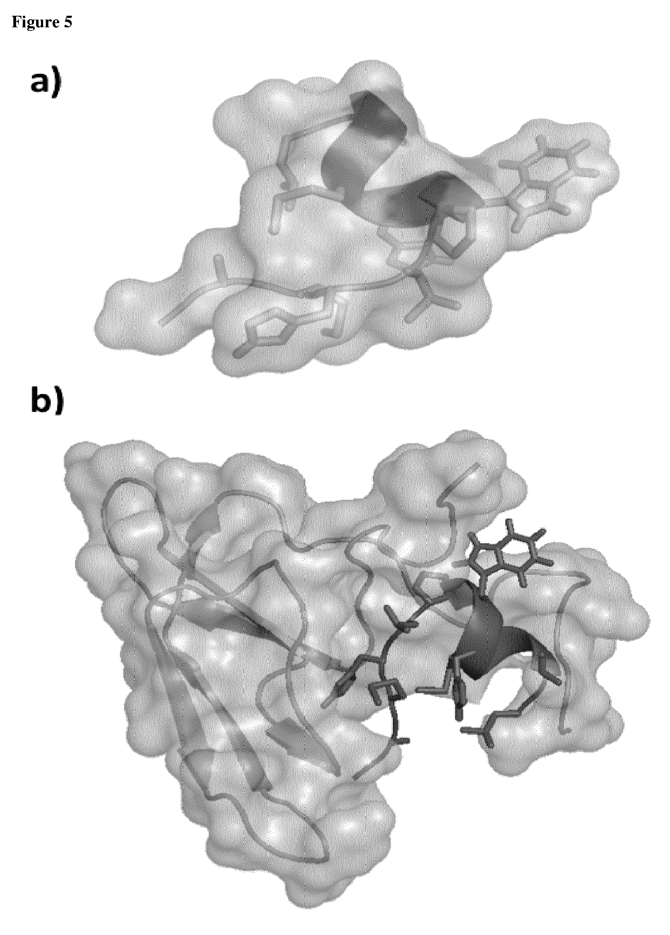

**Figure 5****: *In silico* 3D molecular model of the SEQ ID1 (a) used in this study and in complex (high-resolution rigid body docking prediction (b) with BBI (1BBI.pdb, dark grey)**

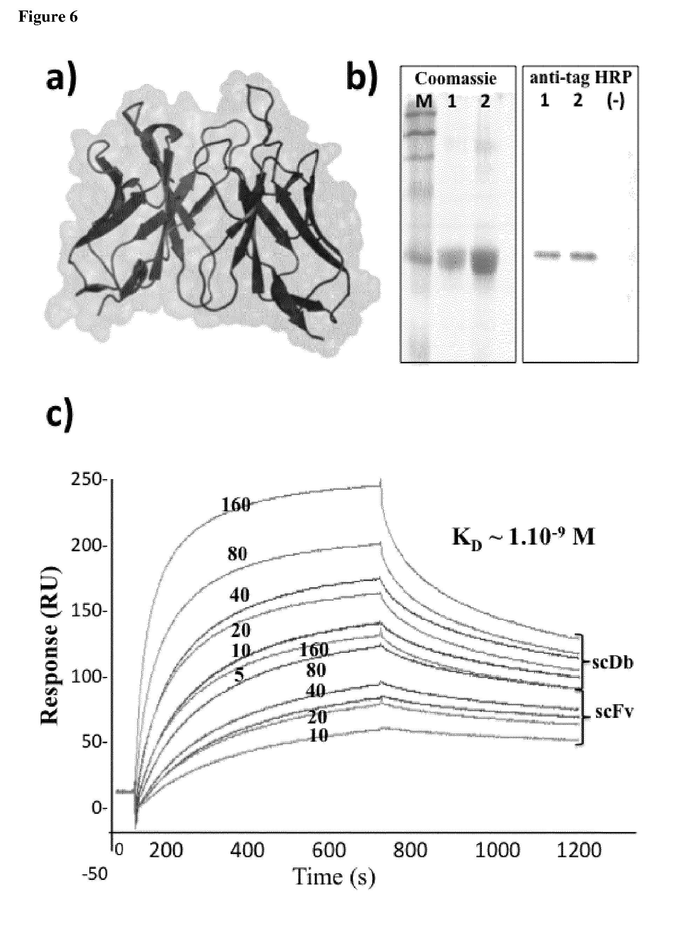

**Figure 6****: Design and characterization of polypeptide structures capable to binding to the soybean BBI and purification thereof. a) Molecular model (Homology** modelling) of the entities comprising SEQ ID 14 and 15 (16, 17, 18, 19, 20, 21) b) Immuno-blot of anti-BBI purified molecules SEQ ID 14 and 15. c) Real time binding interaction (Surface Plasmon resonance, Biacore Technology) to immobilized BBI reveal a nanomolar affinity (scFv refers to single chain antibody fragments and scDb refers to single chain Diabody (a stable and dimeric unit of scFv).
**Figure 7****.** Multivalent probe. A multimeric probe is illustrated.

### Detailed Description of the Drawings

The present invention relates to a number of peptide probes against the soybean Bowman-Birk protease inhibitor (hereafter BBI), i.e., a peptide probe including one of the amino acid sequences SEQ ID NOs:1-21. The term "peptide" used herein refers to a polymer composed of up to 110 amino acid monomers via peptide bond linkage, and optionally includes small proteins having a molecular weight of up to 28kDa. The term probe should be understood to mean a molecule that includes a peptide of the invention, in which the peptide is optionally derivatised with a functional group of a linker suitable for attaching the peptide to another molecule or a support, The probes of the invention comprise one of the SEQ ID NO:s 1-21, especially SEQ ID NO:s 1-13, in monomeric, multimeric (i.e. dimeric, trimeric, etc), multivalent form. The probes may include a linker sequence, typically an amino acid linker, and preferably including the sequence GGGS (SEQ ID NO:22) or GGGSCG (SEQ ID NO:23), or they may be derivatised (functionalised) to facilitate attachment to, for example, a support. The invention also provides a BBI-binding conjugate comprising a probe of the invention covalently bound to a coupling ligand. These peptides of the invention can be prepared by conventional methods, i.e., chemical synthesis or recombinant technology.

When necessary, any of the peptides described herein can be chemically modified to increase their stability. A chemically modified peptide or a peptide analog includes any functional chemical equivalent of the peptide characterized by its increased stability and/or efficacy *in vivo* or *in vitro* in respect of the practice of the invention. The term peptide analog also refers to any amino acid derivative of a peptide as described herein. A peptide analog can be produced by procedures that include, but are not limited to, modifications to side chains, incorporation of unnatural amino acids and/or their derivatives during peptide synthesis and the use of cross-linkers and other methods that impose conformational constraint on the peptides or their analogs. Examples of side chain modifications include modification of amino groups, such as by reductive alkylation by reaction with an aldehyde followed by reduction with NaBH₄; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH₄. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids.

As shown in the Examples below, the peptide probes described herein particularly bind to the BBI, and are capable of purification of BBI from complex protein mixtures such as for example soy whey. For example, a peptide of the invention can be coupled to a support, for example a magnetic bead, and then reacted with a complex protein mixture wherein the peptide of the invention binds BBI present in the mixture and separates the BBI from the other proteins by means of affinity separation.

The term "support" as applied herein means a support for the peptide, and can take a number of different forms including a mobile form (for example magnetic or nonmagnetic beads or particles) or a stationary form (for example a microtitre plate, a filter or membrane, or a stationary phase of a chromatography column). One example of a mobile solid support is beads or particles functionalised with the peptide of the invention. Suitably, the solid support is a magnetic particle, preferably a paramagnetic particle, and ideally a superparamagnetic particle. In the method of the invention, the affinity separation means is suitably separated from the sample by application of a magnetic field. Other types of beads include sepharose or agarose beads. The solid phase may be nano- or micro- scale particles. Preferably, the magnetic particles are nanobeads. The solid support may also take the form of a stationary phase of a chromatographic column. Examples of stationary phases include organic material selected from the group consisting of agarose, dextran, cellulose, chitosan, polyacrylamide, polyacrylate, polystyrene, polyvinyl alcohol, polyethylene glycol, and combinations thereof. Other solid supports applicable for the present invention include plates, for example microtitre plates, and filters, membranes, semi-permeable membranes, a capillary column, a microarrays, and a multiple-well plate.

The solid support may be covalently (chemically) coupled to the peptide probe directly. Coupling of the peptide to the solid phase may be achieved using any method known in the art, for example ethyl-dimethyl-aminopropylcarbodiimide and N-hydroxysuccinimide (EDC/NHS) cross-linking (or PEG-EDC/NHS coupling chemistry) which, for example, couples basic amino acid residues on the peptide (or the linker L) with the solid support (ie. PEI-PEG-NH2), or biotin-biotin binding partner coupling where, for example, the surface of the solid support is functionalised with a biotin binding partner (ie streptavidin or avidin) and peptide probe is biotinylated.

The support preferably comprises a coating of hydrophilic polymer. Examples of hydrophilic polymer include poly(ethylene glycol); copolymers of (2-hydroxyethyl) methacrylate and ethyleneglycol bismethacrylate; and biologically derived polymers such as dextran, albumin, and gelatin. Typically, the hydrophilic polymer has a molecular weight of 1,000 to 1,000,000Da. Suitably, the hydrophilic polymer forms a thin film on the surface of the particle, typically less than 10 nm in thickness. Such oriented films minimise non-specific interactions and very efficiently attach the peptide to the surface of the particle. Methods of attaching a peptide of the invention to the surface of a support will be known to those skilled in the art; for example, a 1,000-100,000Da dextran can be attached to the surface, then activated, prior to covalently coupling the peptide to the activated dextran. Alternatively, a hydrophilic polymer layer that is up to 1,000 nm thick can be synthesised off the particle using highly efficient chemistry such as atomic transfer radiacal polymerisation or the copolymerization of (2-hydroxyethyl) methacrylate and ethyleneglycol bismethacrylate. These coatings have several other purposes. First, they can be tailored to increase the density of the peptides that can be immobilized on the particles which is important for large scale separation processes where large quantities of BBI need to be isolated. These coating can also protect the peptides and particles from harsh chemical and mechanical conditions that the particles are exposed to during the separation process.

The peptide of the invention can be attached to a support in a variety of different ways. For example, the peptides may be synthesised directly on the support, Synthesis techniques include using conventional liquid phase, solidphase synthesis, bacterial synthesis, or any other technologies. Synthesis on the particles has the advantage that it does not require subsequent separation steps and will allow the peptide to be oriented on the surface automatically. In this case, either the conventional FMOC or BOC solid-phase chemistries, i.e., fluorenylmethyloxycarbonyl chloride or benzyloxycarbonyl chloride, respectively, can be used to synthesize the peptide on the surface amino acid by amino acid. The disadvantage of this approach is the peptide will not be completely pure due to the inefficiency of the FMOC and BOC chemistries.

The other approach would be to synthesize the peptide off the affinity separation matrix and then couple it to the materials. In this regard the peptides may comprise a variety of different functional groups available for reaction at the N-terminal, C-terminal, or side groups of the amino acids. Examples of these groups include carboxyl groups, amino groups, thiol groups, etc. Preferably, the peptide is oriented on the surface of the support so a specific group is preferably attached to the N or C terminus of the peptide. This may be achieved by simply adding a thiol through a cystine, which can be reacted with a maleimide group on the particle. Alternatively, it is possible to add a specific functional group such as an azide to the synthesis of the peptide, which would enable a specific and highly efficient azide alkyne huisgen cycloaddition to be performed with an alkyne on the particle.

The term "detectable label" as employed herein means a detectable reporter substance including various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin. Suitably, the reporter molecule is a fluorescent protein (GFP, YFP). Typically, the reporter molecule is an enzyme such as HRP, phosphatase alkaline, enterokinase. In another embodiment, the reporter molecule is a selected from a peptide, a toxin, a fragment from a large protein, and biotin. Typically, the reporter molecule is an affinity tag, as a Strep Tag, Strep Tag II, Avi Tag, His Tag, SA binding peptide, T7 Tag epitope. Alternatively, the reporter molecule is a radioactive compound.

The term "processing stream" as used herein refers to the secondary or incidental product derived from the process of refining a whole legume or oilseed, including an aqueous stream, a solvent stream, or a reconstituted from dried (e.g., spray dried) stream, which includes, for example, an aqueous soy extract stream, an aqueous soymilk extract stream, an aqueous soy whey stream, an aqueous soy molasses stream, an aqueous soy protein concentrate soy molasses stream, an aqueous soy permeate stream, an aqueous tofu whey stream, and additionally includes soy whey protein, for example, in both liquid and dry powder form, that can be recovered as an intermediate product in accordance with the methods disclosed herein.

The term "other proteins" as used herein is defined as including, but not limited to, lunasin, lectins, dehydrins, lipoxygenase, and combinations thereof.

The term "soy whey protein" or "soy whey" as used herein is defined as including proteins soluble at those pHs where soy storage proteins are typically insoluble including, but not limited to, BBI, KTI, lunasin, lipoxygenase, dehydrins, lectins, peptides, and combinations thereof. Soy whey protein may further include storage proteins.

### Materials and selection of anti-BBI binding molecules

Purified BBI and soy extract samples were provided by Solae Denmark A/S (Aarhus, Denmark and from Sigma-Aldrich). Mouse monoclonal HRP labelled anti-M13 antibody was purchased from GE Healthcare (Buckinghamshire, UK). Ultrasensitive streptavidin coupled to peroxidase was from (Sigma Aldrich, Dublin, Ireland). Bovine serum albumin and 1-Step ultra TMB (3,3',5,5,'-tetramentylbenzidine) were from Fisher Scientific (Dublin, Ireland). Protein standards were procured from Alpha-technologies (Wicklow, Ireland). All other reagents were purchased from Sigma-Aldrich Ireland Ltd (Dublin, Ireland) of molecular biology research grade. DNA manipulations were carried out using conventional molecular biology procedures (Sambrook, 1989). All binding and purification experiments were performed at least in duplicate. DNA translations were performed using the online translation tool (Expasy) and sequences aligned using the Clustal program (EBI).

For selection of peptide binders, purified Bowman-Birk Inhibitor (130 µL) of was immobilized in microtiter wells at a concentration of 50 µg/mL in NaHCO3 buffer (0.1M, pH 8.6) overnight at 4 C. After removal of the coating solution, microtiter wells were filled with blocking buffer (2.5 % BSA) for 90 min at 4 0C. The blocking solution was discarded and wells were washed rapidly six times with TBST (TBS + 0.1% Tween-20) before addition of 130 µL of phage particles in TBST buffer to each well for 60 min at room temperature with gentle agitation. Non-binding phage were discarded. Bound phages were subsequently eluted in 100 µL of a low-pH glycine buffer (200 mM, pH 2.2) for 15 min at room temperature. Eluates were immediately neutralized by the addition of 15 µL of Tris buffer (1 M, pH 9.1) and amplified by infection of an early-logarithmic phase culture of *E. coli* in Luria-Bertani medium supplemented with tetracycline (LB-Tetracycline) for 4.5 hours at 37 C. Recombinant phage were purified by double polyethylene glycol (PEG)-NaCl (20 % w/v PEG, 2.5 M NaCl) precipitation. In subsequent rounds of panning, the concentration of immobilised BBI was reduced to 20 µg.mL in the second round and 10 µg/mL in the final 2 rounds while increasing the number of washing steps. After 4 rounds of selection, 32 isolated individual phage clones were selected for purification and submitted to binding experiments by direct solid-phase immuno-assay (ELISA) with all four amplified eluates (phage pool) from the previous rounds of selection and wild type M13 virus acting as a control. Briefly, 100 µL of purified Bowman-Birk Inhibitor (100 µg.mL, in 100 mM NaHCO3) was immobilized onto a 96-well microtiter plate and incubated overnight at 4 0C. Excess target solution was poured out and each well was filled with 2 % BSA in PBS for 90 min at room temperature to block free binding sites. Each well was washed 5 times using PBST (0.1 %) and phage particles in PBST were added to each well. 100 µL of HRP-conjugated anti-M13 pVIII monoclonal antibody (1:2500) was incubated in each well for 60 min. The wells were washed 5 times as before, followed by the addition of 100 µL of TMB substrate to each well. The reaction was terminated by the addition of 30 µL of 1 M sulphuric acid. Absorbance was measured at 450 nm on a microtiter plate reader (Biotek EL808, Winooski, USA).

### Peptide design, synthesis, characterization and binding to BBI

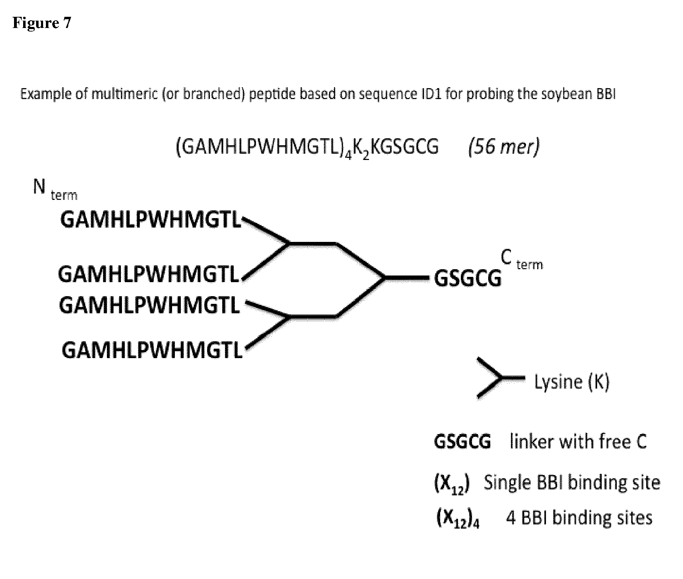

Peptide selection for synthesis (Entelechon, Germany) was based on strong, reproducible and specific reaction of phage clones against BBI in the phage ELISA assays (described previously). The 12-mer linear peptides (SEQ ID 1, 2, 3 and negative control: VAMVLPGVMGTL - SEQ ID NO: 30) were synthesised at >90 % purity. The C-terminus was elongated with a GGGS-CONH2 tail to mimic the GGGS-peptide spacer between the random peptide sequence and the phage protein pIII and to block the negative charge of the carboxyl terminus. The biotinylated peptides and a multimeric structure ("Dendrimer") illustrated in Fig. 7 and comprising four peptides of SEQ ID NO: 1 in a multivalent arrangement were synthesised at >95 % purity, with an additional cysteine-biotin added to the C-terminus - - methods for the synthesis of such branched peptides are described in Leng et al. (Nucleic Acid Research, 2005, Vol. 33, No: 4) and Ota et al (Cancer Research 2002; 62: 1471-1476).. All synthetic peptides were reconstituted in sterile deionised H₂O to a concentration of 2 mg/mL. The ability of biotinylated synthetic peptides (from 0 to 200 µg.mL) to bind to the immobilized purified Bowman-Birk inhibitor, soy pulp and soy whey extracts (and BSA as a negative control) was analyzed in solid phase assays. In this case, the biotinylated synthetic peptides were incubated on BBI coated wells (100 µg.mL) and detected using a streptavidin-HRP conjugate (1:500). Binding and absorbance readings are finally carried out using the TMB substrate as described before.

### Magnetic particle synthesis, functionalisation and characterisation

Superparamagnetic particles were synthesized according to an emulsion solvent evaporation (ESE) procedure developed in our lab (Shang, 2006, Muzard, Platt and Lee, to be published elsewhere). The magnetic properties of the particles were determined using a Quantum Design Superconducting Quantum Interference Device (SQUID). The diameter of the particles was determined (using a Hitachi S-4200 SEM) to be 1 µm with a CV of 20 %. The surface of the iron oxide particle was functionalized with multimeric peptide 1 using Sulfo-SMCC coupling chemistry as follows: 1 mL of 12 mg/mL carboxyl coated particles were washed three times with MES buffer pH 6.0 and 6 mg of both EDC and Sulfo-NHS were added before the sample was sonicated for 30 seconds and incubated for 1 hour at room temperature. The sample was washed three times with carbonate buffer pH 8.2, before 5 % PEI (Mw 1300) in carbonate buffer pH 8.0 was added and the solution was incubated for 2 hours. To couple the PEG to the PEI, the sample was washed with carbonate buffer pH 8.2, 0.6 M K₂SO₄ before 12 mg of Boc-PEG-NHS (Mw 3000) in 1 mL of the high salt carbonate buffer was added to the particles and the sample was then rotated at 50 °C for 12 hours. To couple the microparticles to the selected peptide the Boc groups were removed by incubating the sample in a 50 % v/v TFA solution for 5 minutes. The suspension was then washed three times with PBS pH 7.5 before a 4 mg/mL solution of Sulfo-SMCC in PBS was added to the particles for 40 minutes. The solution was then resuspended in 1 mL PBST pH 6.75 and immediately added to the multimeric peptide 1 (~1 mg) overnight. Finally, the multimeric peptide linked magnetic particles were washed five times with PBST and stored at 4 °C before use. Peptide immobilization on particles was analysed using a MALDI TOF mass spectrometer (SAI, UK) operating at 20 kV in linear mode with MS grade sinapinic acid as a matrix. All zeta potential measurements were performed using a Malvern Zetasizer Nano ZS, using the DTS-1060 cells. 20 mL of particles were placed into a 1.5 mL centrifuge tube and washed three times with 1ml of the required buffer before being resuspended in appropriate buffer and pH.

### Real-time binding analysis (Surface Plasmon Resonance)

The BIAcore T100 instrument and all the reagents for analysis were obtained from GE Healthcare Ltd. (Buckinghamshire, UK). Soluble purified BBI was immobilized (approximatively 500 RU) on a carboxymethyldextran CM5 sensor chip activated with a 1:1 mix of N-hydroxysuccinimide (50 mM) and N-ethyl-N-(dimethylaminopropyl)-carbodiimide (200 mM) by a 7 min pulse. Purified anti-BBI entities were then passed over the BBI surface in HBS-EP buffer [0.01 mM Hepes (pH 7.4), 0.15 mM NaCl, 0.005% polysorbate 20 (v/v)] at a flow rate of 20 µL.min-1 at 25 °C. Glycine-HCl (10 mM, pH 2.0) was injected for 30 sec at 20 µL.min-1 to regenerate the sensor chip between successive samples. Kinetic constants (kₒₙ, k_{off}) were deduced from the analysis of association and dissociation rates at four different concentrations, ranging from 5 to 160 µg/mL-1. kₒₙ refers to the kinetic of binding association rate, and k_{off} refers to the dissociation rate - KD (affinity) corresponds to the ratio of the these parameters. The dissociation constant KD was calculated from KD = koff/kon. Sensorgrams were analyzed using biaevaluation software. Guidelines for accurate kinetic analysis were employed, and data were double-referenced as described in in the litterature. All experiments were carried out in triplicate.

### Magnetic based purification of BBI from crude soy samples

Soy crude protein extracts (~100 mL) were incubated for 16 h at 20 °C with 1 mL functionalized magnetic particles (10 mg.mL). After extensive washing with PBST until A280 reached 0.001, bound proteins were eluted in fractions of 100 µL of pH 2.2 Glycine (100 mM), with buffer and fractions > 0.1 being submitted to a MALDI-TOF assay, SDS-PAGE analysis and enzymatic assays. Identification of the stained proteins was performed by mass spectrometry. Bands of interest were excised out of the SDS-PAGE gel and submitted to tryptic digestion, according to the protocol described by Shevchenko et al. (1996). The resulting tryptic peptides were desalted and concentrated using ZipTips (Eppendorf, Hamburg, Germany) according to the manufacturer's instructions. Peptides were re-suspended in 12 µl of 0.1 % formic acid. Each sample was loaded onto a Biobasic C18 Picofrit TM column (100 mm length, 75 mm ID) and was separated by a 72 min reverse phase increasing acetonitrile gradient (0-50 % acetonitrile for 50 min) at a flow rate of 30 nL. min-1 on an Dionex Ultimate 3000 chromatography system incorporating an auto-sampler. Nano-ESI LC-MS/MS was carried out on a Thermo Scientific LTQ ORBITRAP XL mass spectrometer. The instrument was operated in positive ion mode with a capillary temperature of 200 °C, a capillary voltage of 9 V, a tube lens voltage of 100 V and with a potential of 1800 V applied to the frit. All data was acquired with the mass spectrometer operating in automatic data dependent switching mode. A high resolution MS scan (300-2000 Dalton) was performed using the Orbitrap, followed by MS/MS analysis of the 5 most intense ions using the ion trap. PEAKS Studio version 5.2 (Bioinformatic Solutions, Waterloo, ON Canada) was used for the simultaneous database matching to the NCBI non-redundant database, taxonomy *Glycine Max* (soybean) and de novo sequencing of good-quality spectra. Up to three missed cleavages were allowed and cysteine carbamidomethylation and methionine oxidation were set as variable modifications. Precursor tolerance was set at 10 ppm, fragment tolerance at 0.5 Da.

### Enzymatic assays

Inhibition of trypsin activity was measured on eluted fractions at 25 °C in 0.4 M Tris-C1, 0.01 mM CaCl, pH 8.1, and with 1 mM (p-tolylsulfonyl) -L- arginine ethyl ester, as substrate (Hummel, 1959). Inhibition of the chymotrypsin activity was determined at 25 °C in 0.04 M Tris-C1, 0.05 M CaCl, pH 7.8, with 0.5 mM benzoyl-L-tyrosine ethyl ester, as substrate (Hummel, 1959). Readings were recorded every 20 seconds at 256 nm in a UV/VIS NIR spectrophotometer (Varian Cary model 6000i, Agilent Technologies, Palo Alto, USA).

This invention has developed peptide structure molecular entities/probes against the BBI allowing the detection and purification of the Bowman-Birk inhibitor from crude soy whey mixtures. These peptides, when grafted onto superparamagnetic particles and incubated with soy whey samples will allow purification of BBI from complex mixtures by the application of an external magnetic field.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A peptide probe against the soybean Bowman-Birk Inhibitor (BBI), comprising an amino acid sequence selected from the group consisting of:
GAMHLPWHMGTL (SEQ ID NO:1);
KFLGPLNHRDRH (SEQ ID NO:2),
ANYFLPPVLSSS (SEQ ID NO:3),
GAKHLPRHKGPL (SEQ ID NO:4),
GAMHLLRHMGPL (SEQ ID NO:5)
TGHQSPGAYAAH (SEQ ID NO:6),
FTTEDFYNHFPH (SEQ ID NO:7),
APQDNTIMPSPT (SEQ ID NO:8),
MPAVMSSAQVPR (SEQ ID NO:9),
HDFRHSGHNHPP (SEQ ID NO:10)
DHASHWMVKRGV (SEQ ID NO:11),
SSMYTYHRTGDF (SEQ ID NO:12),
CGLPPHRTC (SEQ ID NO:13),
EVQLQQSGAELVKPGASVKLSCTGSGFNIKDNNIHWLRQRPEQGLEWIG KIDPANGDTEYGPKLQGKATITSDTSSNTAYLQLNSLTSEDTAVYYCAT DLYSKPVWFAYWGQGTLVTVSA (SEQ ID NO: 14),
DVLMSQAPLSLPVSLGDQASISCRSSQNIVHSNGNTYLEWYLQKPGQSP KLLIYKVSNRFSGVPDRFSGRGSGTDFTLKISRVEAEDLGVYYCFQGSHV PWTFGGGTKLEIK (SEQ ID NO: 15),
FNIKDNNIHWLR (SEQ ID NO: 16),
GDTEYGPKLQ (SEQ ID NO: 17),
VWFAYWGQGTLVT (SEQ ID NO: 18),
QNIVHSNGNTYL (SEQ ID NO: 19),
VSNRFSGVPD (SEQ ID NO: 20),
WTFGGGTKLEIK (SEQ ID NO: 21)

2. A peptide probe of claim 1, wherein the peptide includes the amino acid sequence of any of SEQ ID NO: 1 to 13.

3. A peptide probe of claim 1, wherein the peptide includes the amino acid sequence of SEQ ID NO: 2, 4, 5, 7, 8 and 10.

4. A peptide probe of claim 1, wherein the peptide includes the amino acid sequence of SEQ ID NO: 1.

5. A multimeric peptide probe (P)n, wherein P is a peptide probe of Claim 2, 3, or 4 and n is a number from 2 to 10.

6. A peptide probe of any of claims 1 to 4, or a multimeric peptide probe of claim 5, derivatised with (a) a linker L or functional group suitable for linking the peptide probe or the multimeric peptide probe to another molecule or a support, or (b) a detectable label

7. A derivatised peptide probe or multimeric peptide probe as claimed in Claim 6 in which the linker L is a linker peptide including an amino acid sequence GGGS (SEQ ID NO: 22) or GGGSCG (SEQ ID NO: 23), in which the derivatised peptide probe or multimeric peptide probe is selected from the group consisting of SEQ ID NOS: 24 to 29.

8. An affinity separation matrix comprising a support having a peptide probe of any of Claims 1 to 4, a multimeric peptide probe of Claim 5, or a derivatised peptide probe or multimeric peptide probe of Claim 6 or 7 immobilized thereon, in which the support is selected from a plate, a membrane, a bead, a nano and/or micro sized particle, a magnetic particle, and a stationary phase of a chromatography column.

9. An affinity separation matrix as claimed in Claim 8 in which the support is coated with a thin film of hydrophilic polymer having a molecular weight of 1,000 to 10,000Da..

10. An affinity separation matrix as claimed in Claim 8 or 9 in which the hydrophilic polymer is poly(ethylene glycol).

11. A method of generating an affinity separation matrix as claimed in any of Claims 8 to 10 and comprising a step of providing a peptide probe of any of Claims 1 to 4, a multimeric peptide probe of Claim 5, or a derivatised peptide probe or multimeric peptide probe of Claim 6 or 7, on a surface of the support.

12. A method of generating an affinity separation matrix as claimed in any of Claims 8 to 10 and comprising a step of providing a derivatised peptide probe of Claim 6, in which the peptide is derivatised with a functional group, and coupling the derivatised peptide probe to a support.

13. An antibody or antibody fragment having a variable heavy (VH) region, the VH region having an amino acid sequence selected from the group consisting of SEQ ID NO: 14, 16, 17 and 18.

14. An antibody or antibody fragment having a variable heavy (VL) region, the VL region having an amino acid sequence selected from the group consisting of SEQ ID NO: 15, 19, 20 and 21.

15. Use of a peptide, multimeric peptide, or derivatised peptide or multimeric peptide of any of Claims 1 to 7, for the detection, separation or purification of BBI protein.


SEQUENCE LISTING

<110>  UNIVERSITY COLLEGE DUBLIN, NATIONAL UNIVERSITY OF IRELAND,
       DUBLIN

<120>  Bowman-Birk protease inhibitor (BBI) binding peptides, and uses
       thereof in the separation, detection or purification of BBI

<130>  P10780EP00

<160>  30

<170>  PatentIn version 3.3

<210>  1
<211>  12
<212>  PRT
<213>  Artificial

<220>
<223>  synthetic

<400>  1

Gly Ala Met His Leu Pro Trp His Met Gly Thr Leu
1               5                   10


<210>  2
<211>  12
<212>  PRT
<213>  Artificial

<220>
<223>  synthetic

<400>  2

Lys Phe Leu Gly Pro Leu Asn His Arg Asp Arg His
1               5                   10


<210>  3
<211>  12
<212>  PRT
<213>  Artificial

<220>
<223>  Synthetic

<400>  3

Ala Asn Tyr Phe Leu Pro Pro Val Leu Ser Ser Ser
1               5                   10


<210>  4
<211>  12
<212>  PRT
<213>  Artificial

<220>
<223>  Synthetic

<400>  4

Gly Ala Lys His Leu Pro Arg His Lys Gly Pro Leu
1               5                   10

<210>  5
<211>  12
<212>  PRT
<213>  Artificial

<220>
<223>  Synthetic

<400>  5

Gly Ala Met His Leu Leu Arg His Met Gly Pro Leu
1               5                   10

<210>  6
<211>  12
<212>  PRT
<213>  Artificial

<220>
<223>  Synthetic

<400>  6

Thr Gly His Gln Ser Pro Gly Ala Tyr Ala Ala His
1               5                   10

<210>  7
<211>  12
<212>  PRT
<213>  Artificial

<220>
<223>  Synthetic

<400>  7

Phe Thr Thr Glu Asp Phe Tyr Asn His Phe Pro His
1               5                   10

<210>  8
<211>  12
<212>  PRT
<213>  Artificial

<220>
<223>  Synthetic

<400>  8

Ala Pro Gln Asp Asn Thr Ile Met Pro Ser Pro Thr
1               5                   10

<210>  9
<211>  12
<212>  PRT
<213>  Artificial

<220>
<223>  Synthetic

<400>  9

Met Pro Ala Val Met Ser Ser Ala Gln Val Pro Arg
1               5                   10


<210>  10
<211>  12
<212>  PRT
<213>  Artificial

<220>
<223>  Synthetic

<400>  10

His Asp Phe Arg His Ser Gly His Asn His Pro Pro
1               5                   10


<210>  11
<211>  12
<212>  PRT
<213>  Artificial

<220>
<223>  Synthetic

<400>  11

Asp His Ala Ser His Trp Met Val Lys Arg Gly Val
1               5                   10


<210>  12
<211>  12
<212>  PRT
<213>  Artificial

<220>
<223>  Synthetic

<400>  12

Ser Ser Met Tyr Thr Tyr His Arg Thr Gly Asp Phe
1               5                   10


<210>  13
<211>  9
<212>  PRT
<213>  Artificial

<220>
<223>  Synthetic

<400>  13

Cys Gly Leu Pro Pro His Arg Thr Cys
1               5

<210>  14
<211>  120
<212>  PRT
<213>  Artificial

<220>
<223>  Synthetic

<400>  14

Glu Val Gln Leu Gln Gln Ser Gly Ala Glu Leu Val Lys Pro Gly Ala
1               5                   10                  15

Ser Val Lys Leu Ser Cys Thr Gly Ser Gly Phe Asn Ile Lys Asp Asn
            20                  25                  30

Asn Ile His Trp Leu Arg Gln Arg Pro Glu Gln Gly Leu Glu Trp Ile
        35                  40                  45

Gly Lys Ile Asp Pro Ala Asn Gly Asp Thr Glu Tyr Gly Pro Lys Leu
    50                  55                  60

Gln Gly Lys Ala Thr Ile Thr Ser Asp Thr Ser Ser Asn Thr Ala Tyr
65                  70                  75                  80

Leu Gln Leu Asn Ser Leu Thr Ser Glu Asp Thr Ala Val Tyr Tyr Cys
                85                  90                  95

Ala Thr Asp Leu Tyr Ser Lys Pro Val Trp Phe Ala Tyr Trp Gly Gln
            100                 105                 110

Gly Thr Leu Val Thr Val Ser Ala
        115                 120


<210>  15
<211>  112
<212>  PRT
<213>  Artificial

<220>
<223>  Synthetic

<400>  15

Asp Val Leu Met Ser Gln Ala Pro Leu Ser Leu Pro Val Ser Leu Gly
1               5                   10                  15

Asp Gln Ala Ser Ile Ser Cys Arg Ser Ser Gln Asn Ile Val His Ser
            20                  25                  30

Asn Gly Asn Thr Tyr Leu Glu Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Arg Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Val Pro Trp Thr Phe Gly Gly Gly Thr Lys Leu Glu Ile Lys
            100                 105                 110

<210>  16
<211>  12
<212>  PRT
<213>  Artificial

<220>
<223>  Synthetic

<400>  16

Phe Asn Ile Lys Asp Asn Asn Ile His Trp Leu Arg
1               5                   10

<210>  17
<211>  10
<212>  PRT
<213>  Artificial

<220>
<223>  Synthetic

<400>  17

Gly Asp Thr Glu Tyr Gly Pro Lys Leu Gln
1               5                   10

<210>  18
<211>  13
<212>  PRT
<213>  Artificial

<220>
<223>  Synthetic

<400>  18

Val Trp Phe Ala Tyr Trp Gly Gln Gly Thr Leu Val Thr
1               5                   10

<210>  19
<211>  12
<212>  PRT
<213>  Artificial

<220>
<223>  Synthetic

<400>  19

Gln Asn Ile Val His Ser Asn Gly Asn Thr Tyr Leu
1               5                   10


<210>  20
<211>  10
<212>  PRT
<213>  Artificial

<220>
<223>  Synthetic

<400>  20

Val Ser Asn Arg Phe Ser Gly Val Pro Asp
1               5                   10


<210>  21
<211>  12
<212>  PRT
<213>  Artificial

<220>
<223>  Synthetic

<400>  21

Trp Thr Phe Gly Gly Gly Thr Lys Leu Glu Ile Lys
1               5                   10


<210>  22
<211>  4
<212>  PRT
<213>  Artificial

<220>
<223>  linker peptide

<400>  22

Gly Gly Gly Ser
1


<210>  23
<211>  6
<212>  PRT
<213>  Artificial

<220>
<223>  Synthetic

<400>  23

Gly Gly Gly Ser Cys Gly
1               5


<210>  24
<211>  16

<212>  PRT
<213>  Artificial

<220>
<223>  Synthetic

<400>  24

Gly Ala Met His Leu Pro Trp His Met Gly Thr Leu Gly Gly Gly Ser
1               5                   10                  15


<210>  25
<211>  16
<212>  PRT
<213>  Artificial

<220>
<223>  Synthetic

<400>  25

Ala Asn Tyr Phe Leu Pro Pro Val Leu Ser Ser Ser Gly Gly Gly Ser
1               5                   10                  15


<210>  26
<211>  16
<212>  PRT
<213>  Artificial

<220>
<223>  Synthetic

<400>  26

Lys Phe Leu Gly Pro Leu Asn His Arg Asp Arg His Gly Gly Gly Ser
1               5                   10                  15


<210>  27
<211>  18
<212>  PRT
<213>  Artificial

<220>
<223>  Synthetic

<400>  27

Gly Ala Met His Leu Pro Trp His Met Gly Thr Leu Gly Gly Gly Ser
1               5                   10                  15


Cys Gly


<210>  28
<211>  18
<212>  PRT
<213>  Artificial

<220>
<223>  Synthetic

<400>  28

Ala Asn Tyr Phe Leu Pro Pro Val Leu Ser Ser Ser Gly Gly Gly Ser
1               5                   10                  15

Cys Gly

<210>  29
<211>  18
<212>  PRT
<213>  Artificial

<220>
<223>  Synthetic

<400>  29

Lys Phe Leu Gly Pro Leu Asn His Arg Asp Arg His Gly Gly Gly Ser
1               5                   10                  15

Cys Gly

<210>  30
<211>  12
<212>  PRT
<213>  Artificial

<220>
<223>  Synthetic

<400>  30

Val Ala Met Val Leu Pro Gly Val Met Gly Thr Leu
1               5                   10